(19) Europäisches Patentamt
European Patent Office
Office européen des brevets

(11) Publication number: **0 121 191**
**A2**

(12) # EUROPEAN PATENT APPLICATION

(21) Application number: 84103211.3

(22) Date of filing: 23.03.84

(51) Int. Cl.³: C 12 Q 1/28
G 01 N 33/52

(30) Priority: 04.04.83 US 481631

(43) Date of publication of application:
10.10.84 Bulletin 84/41

(84) Designated Contracting States:
DE FR GB

(71) Applicant: MILES LABORATORIES, INC.
1127 Myrtle Street
Elkhart Indiana 46514(US)

(72) Inventor: Gantzer, Mary Lou
25723 Kiser Court
Elkhart, IN 46514(US)

(74) Representative: Adrian, Albert, Dr. et al,
c/o BAYER AG Konzernverwaltung RP Patentabteilung
D-5090 Leverkusen 1, Bayerwerk(DE)

(54) **Analytical test composition, device, method for its preparation and method for the determination of peroxidatively active substances.**

(57) A test composition, device and method for determining a peroxidatively active substance in a test sample, as well as a method for preparing the device, are disclosed. The composition comprises an organic hydroperoxide and an indicator capable of providing a detectable response in the presence of the organic hydroperoxide and peroxidatively active substance, wherein the organic hydroperoxide is tetralin hydroperoxide. The device comprises a carrier matrix incorporated with the composition, and the method comprises contacting a test sample with the device and observing a detectable response in the device. A method for preparing the device comprises preparing a solution of the composition ingredients, wetting the carrier matrix therewith and drying the wetted matrix.

EP 0 121 191 A2

# ANALYTICAL TEST COMPOSITION, DEVICE, METHOD FOR ITS PREPARATION AND METHOD FOR THE DETERMINATION OF PEROXIDATIVELY ACTIVE SUBSTANCES

## *BACKGROUND OF THE INVENTION*

### *1. Field of the Invention*

This invention relates generally to analytical tests for the determination of peroxidatively active substances in test samples, and particularly to an improved test composition and device for such determinations having enhanced storage stability, as well as to a method for making and using the improved composition and device.

Many analytical methods are available for detecting the presence of peroxidatively active substances in biological samples such as urine, fecal suspensions and gastrointestinal contents. Hemoglobin and its derivatives are typical examples of such "peroxidatively active" substances because they behave in a manner similar to the enzyme peroxidase. Such substances have also been referred to as *pseudoperoxidases*, i.e., enzyme-like in that they catalyze the redox reaction between peroxides and such indicator compounds as benzidine, *o*-tolidine, 3,3',5,5'-tetramethylbenzidine, 2,7-diaminofluorene or similar

substances, thereby producing a detectable response such as a color change. Most methods for determining the presence of occult blood in test samples therefore rely on this *pseudoperoxidase* activity.

2. *Background Art*

Analytical test methods have evolved over the years which rely on enzyme-like catalysis of the peroxidative oxidation of colorforming indicators. These include wet chemical or solution procedures and the so-called "dip-and-read" type, reagent-bearing strip devices. Of the former, a typical example is set forth in Richard M. Henry, et al., *Clinical Chemistry Principles and Techniques*, 2nd ed., (Hagerstown, Maryland: Harper and Row, 1974), pp. 1124-1125. This procedure involves the use of glacial acetic acid (buffer), diphenylamine (indicator) and hydrogen peroxide. While such wet methods have proven analytical utility, they also have shortcomings, such as poor reagent stability and inadequate sensitivity.

Another method for the determination of peroxidatively active substances, and one presently preferred by most clinical assayists, employs the "dip-and-read", solid phase reagent strip device. Typical of such devices are those commercially available from the Ames Division of Miles Laboratories, Inc. under the trademark HEMASTIX®. They comprise, in essence, a porous paper matrix affixed to a plastic strip or handle. The matrix is impregnated with a buffered mixture of an organic hydroperoxide, for example, cumene hydroperoxide, and an indicator compound. Upon immersion in a liquid containing an

analyte such as hemoglobin, myoglobin, erythrocytes or other *pseudoperoxidase*, a blue color develops in the matrix, the intensity of which is proportional to the concentration of the peroxidatively active substance in the sample. Thus, by comparing the color developed in the matrix to a standard color chart, the assayist can determine, on a semiquantitative basis, the amount of analyte present in the sample.

Reagent strips possess a number of advantages over wet chemistry methods, for example, greater ease of use because neither the preparation of reagents nor attendant apparatus is required, and greater comparative stability of reagents because of the dry, solid reagent state, resulting in improved accuracy, sensitivity and economy. However, a serious disadvantage of many conventional, presently-available reagent strip test devices has been limited "shelf-life", i.e., a lack of storage stability over prolonged periods following manufacture, resulting in markedly decreased reactivity to the presence of peroxidatively active analytes when the devices are used. Thus, because analytical tools such as reagent strips usually are not used immediately after manufacture, but stored for varying periods of time before use, and because too long a period between manufacture and use of conventional reagent strips can result in a severe loss of reactivity, leading to false negative test results, enhanced shelf-life can be a marked asset: the better the shelf-life, the more dependable the analytical results.

Conventional solid phase reagent strip devices for determining peroxidatively active substances, e.g., occult blood or hemoglobin in urine, typically

utilize as an indicator system the porphyrin-catalyzed oxidation of a benzidine-type indicator, for example, *o*-tolidine or 3,3',5,5'-tetramethylbenzidine, by an organic hydroperoxide, such as cumene hydroperoxide. Such conventional test strips, however, are known to be particularly prone to loss of reactivity during prolonged storage, or storage at elevated temperatures -- a phenomenon which is believed to be due either to volatility or chemical degradation of one or more reagent ingredients of the strip. Not only have substantial losses of reactivity been observed in such conventional reagent strips following storage at ambient temperatures, but those losses appear to be substantially accentuated, and the rate of loss accelerated, by storage at elevated temperatures. Possible explanations for the losses of reactivity in reagent strips are: (1) key ingredient(s) of the reagent composition decompose or volatilize, so that the level of ingredient(s) falls below the minimum level necessary to maintain adequate reactivity; and (2) two or more ingredients in the strip interact deleteriously, producing one or more new species which are unreactive or inhibitory.

Attempts to stabilize the reactivity of reagent compositions, and solid phase strip devices made therefrom for determining peroxidatively active substances, have followed various lines of approach. For example, U.S. Patent No. 3,092,463 to Adams, Jr. et al., discloses an improved test composition and device for detecting occult blood in body fluids. The composition comprises an organic hydroperoxide encapsulated or entrapped in microspherical bubbles of a colloid substance, an indicator or dye precursor capable of accepting transfer of oxygen from the

organic hydroperoxide to produce a color response induced by the catalytic action of the prosthetic group of hemoglobin, and a buffer for maintaining the pH of the substance being tested within the range of from 4 to 6.5. This patent discloses that the colloid substance, for example, polyvinyl alcohol, gelatin, gum arabic or carboxy vinyl polymer, used to encapsulate or entrap the hydroperoxide, can provide stabilization of the reactivity of preferred embodiments of the test device produced from the composition even after 300 hours storage at 75°C, whereas similar devices prepared without encapsulation of the hydroperoxide lost reactivity after 24 hours at 50°C.

Other disclosures of stabilized test compositions and devices include the approach of U.S. Patent No. 3,252,762 to Adams, Jr. et al., wherein the organic hydroperoxide used is encapsulated in a colloidal material such as gelatin which is hardened by fixing with a dialdehyde polysaccharide. Such compositions, containing a hydroperoxide so encapsulated, a suitable indicator and a buffer, are said to exhibit enhanced stability under various adverse temperature conditions.

Still further disclosed attempts at stabilization of such reagent strip devices include a recitation in *Chemical Abstracts*, Vol. 85, p. 186 (1976), describing a two-dip method for preparing reagent strips containing *o*-tolidine and phenylisopropyl hydroperoxide. This disclosure reports making a solution of the indicator (*o*-tolidine • 2HCl) and polyvinylpyrrolidone in ethanol. To this solution was added a small amount of surfactant and enough citrate buffer to provide a pH of 3.7, whereafter filter paper strips impregnated with ethyl

cellulose were dipped in this solution and dried. The impregnated filter paper was subsequently dipped into a second solution containing 1,4-diazabicyclo [2,2,2]octane, phenylisopropyl hydroperoxide and polyvinyl pyrrolidone, dissolved in an ethanol-toluene mixture. The thrust of this work was to stabilize the peroxide and indicator combination through the use of the bicyclooctane derivative and the polyvinylpyrrolidone.

A similar method is disclosed in U.S. Patent No. 3,853,471. This patent discloses the use of phosphoric or phosphonic acid amides where the substituent amido groups are primarily N-morpholine radicals.

Other approaches to stabilize reagent compositions include that of U.S. Patent No. 4,071,317, wherein various diluent compounds, such as a mixture of dimethyl sulfone and N,N-dimethyl formamide, are employed in addition to cumene hydroperoxide and an indicator; of U.S. Patent No. 4,071,318 (use of borate esters); and of U.S. Patent No. 4,071,321 (use of both diluents and borate esters).

Another reference which is of interest to these general concepts is U.S. Patent No. 3,236,850, directed toward stabilizing organic hydroperoxides used as catalysts and oxidizing agents. This reference discloses the use of primary, secondary or tertiary amine salts with organic peroxides, and is not directed to stability problems of solid phase reagent test strip devices.

Despite the inherent analytical advantages of solid phase reagent strip devices over wet chemistry procedures, and the foregoing exemplary advances in the art of stabilizing the reactivity of such strip devices, the stability characteristics of the

latter, particularly in the case of devices for the determination of peroxidatively active substances, are in need of still further improvement. Whereas the properties of current solid phase, state-of-the-art compositions and devices for determining peroxidatively active substances have been greatly enhanced over those of wet chemical methods, and over those including no stability-enhancement techniques, it would nonetheless be greatly advantageous if such strips could be made even more stable during prolonged storage, and thus more sensitive to peroxidatively active anaytes following such storage, without the need for the addition of chemical substances, isolation of reagents by encapsulation, or similar relatively complicated and expensive treatments of such compositions and devices. For example, it would be a great advance in the art to provide a suitable, direct substitute for the well-known organic hydroperoxides which are conventionally used in solid phase test compositions and devices, and one which would render the reagent system of these compositions and devices more stable during long-term storage. Conventionally-used hydroperoxides include, for example, cumene hydroperoxide, *t*-butyl hydroperoxide, diisopropylbenzene hydroperoxide, 2,5-dimethyl-hexane-2,5-dihydroperoxide and paramenthane hydroperoxide.

Any substitute selected to replace conventional organic hydroperoxides, such as those previously listed in compositions and devices for determining a peroxidatively active analyte, must not only be capable of participating similarly in the redox reaction with the analyte, in the presence of the conventional indicator systems used, to produce a detectable response such as a color change or change in the amount of light absorbed or reflected by the test composition,

but also must exhibit such reactivity to an extent comparable to that of those conventional hydroperoxides. C. Chen and C. C. Lin, *Biochim. Biophys. Acta*, 170 (1968), pp. 366-374, disclose that, in solution experiments, tetralin hydroperoxide showed some catalytic activity when incubated with a 100 milligrams per deciliter (mg/dl) concentration of hemoglobin for one hour at 37°C, in its conversion to tetralol. This article also discloses that tetralin hydroperoxide is stable under normal solution conditions and in the presence of a variety of iron compounds, except hemoglobin. Accordingly, the advantage of its use over conventional organic hydroperoxides, at least in wet chemistry procedures, would appear to be evident. However, so far as is presently known, the literature lacks any suggestion as to the participation of tetralin hydroperoxide, in the solid phase, in the catalytic reaction with peroxidatively active substances such as hemoglobin. Indeed, the authors describe the catalytic activity of this hydroperoxide with hemoglobin in solution as being only some 36% of the activity obtainable with an active liver enzyme, reduced nicotinamide adenine dinucleotide phosphate (NADPH). This is an activity level well below what would be considered adequate for use in solid phase reagent assays for peroxidatively active substances, wherein analyte concentrations as low as 0.045 mg/dl can be expected. By comparison with the activity shown by such conventionally used compounds as cumene hydroperoxide, therefore, this literature contains no suggestion that tetralin hydroperoxide would function adequately in the catalytic reaction with a benzidine-type indicator system as typically used in conventional solid phase test devices.

It has now been postulated that the frequently-observed losses of reactivity, leading to a lack of storage stability, or "shelf-life", of conventional solid phase reagent compositions and devices for

determining peroxidatively active substances, may be attributable to loss and/or chemical degradation of the organic hydroperoxide used in the reagent strip. Such loss or degradation could occur, for example, from decomposition or volatilization of the hydroperoxide, or chemical interaction with other strip constituents. However, it is now believed that degradation due to decomposition or deleterious interaction may account for most reactivity losses. The mechanism causing such degradation is at the present unknown.

*SUMMARY OF THE INVENTION*

It has now been discovered, and the present invention is based upon this discovery, that present "state-of-the-art", conventional solid phase reagent systems for determining peroxidatively active substances can be improved substantially, and the aforementioned stability problems of such conventional systems largely overcome, through the advantageous inclusion of tetralin hydroperoxide in such reagent systems. According to the invention, tetralin hydroperoxide is used as an oxidizing agent in place of one or more of those organic hydroperoxides typically used in the art, such as cumene hydroperoxide. This direct substitution has been found to result in enhanced economy and reliability for the composition of the invention by comparison with stabilized compositions employing such techniques as isolation of reagents, chemical additives, or the like. Moreover, tetralin hydroperoxide appears to unexpectedly enable the system to exhibit, in the solid phase, sufficient sensitivity to the presence of peroxidatively active substances to make it an advantageous analytical tool, with performance characteristics comparable to that of conventional compositions employing cumene hydroperoxide. Detection of hemoglobin in urine at concentrations as low as 0.045 milligrams per deciliter (mg/dl) has been accomplished by the invention, despite the known reaction characteristics of tetralin hydroperoxide in the presence of hemoglobin, which, in view of the solution experiments aforedescribed, indicate that this hydroperoxide would be unsuitable for use.

Unlike conventional analytical reagent systems for determining peroxidatively active substances which generally comprise an organic hydroperoxide and a benzidine-type redox indicator, the present invention provides an improved, solid phase, stabilized analytical composition for determining such a substance in a test sample. The composition is improved by the use of tetralin hydroperoxide, and an indicator capable of providing a detectable response in the presence of the hydroperoxide and the peroxidatively active substance, the latter catalyzing or otherwise participating in a reaction between the hydroperoxide and the indicator, which in turn produces a color or other detectable response whose intensity is indicative of the concentration of the analyte.

The present invention also provides an improved, solid phase analytical device, which, in a preferred embodiment, comprises a carrier matrix incorporated with the improved composition of the invention. It is believed that the overall effect of the novel combination of tetralin hydroperoxide with other, largely conventional ingredients in the composition, enables the reactivity of the composition, and the device incorporating it, to be significantly stabilized, particularly over long periods of storage at both ambient and elevated temperatures. This provides advantageously enhanced "shelf-life" by comparison with conventional compositions and devices. Moreover, the composition and device of the invention have excellent

sensitivity to the presence of a peroxidatively active analyte in a test sample.

In addition, a method for using the analytical device of the invention is provided, as well as a method for making it. In a preferred embodiment, the device is used by immersing it into a liquid test sample under analysis and observing the detectable response, such as a color change, produced therein. Preferably, the method for making the device comprises incorporating a carrier matrix, for example a bibulous paper, with a solution or suspension of the ingredients of the composition.

*DETAILED DESCRIPTION OF THE INVENTION*

The tetralin hydroperoxide used in the present invention is either available commercially or can be readily prepared from commercially available materials, using largely conventional organic synthesis techniques. See, for example, the procedure described in H.B. Knight and D. Swern, *Org. Syn.* 34 (1954), p. 90.

Thus, tetralin hydroperoxide can be prepared, as described in the Example, *infra*, via air oxidation of tetralin:

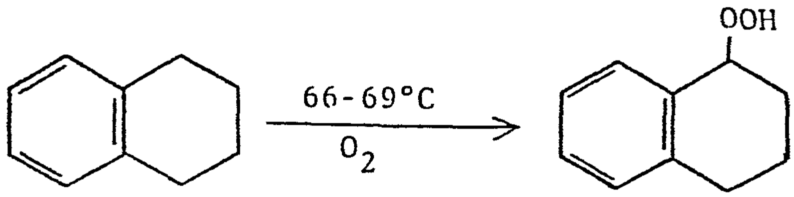


tetralin    tetralin hydroperoxide

In the foregoing synthesis, excess tetralin can be removed by distillation at reduced pressure and the product recrystallized from toluene.

The amount of tetralin hydroperoxide used in the composition and device is not of critical importance to the achievement of the advantages of the invention, and is a matter of routine choice for one skilled in the art based upon the analyte determined and the level of sensitivity desired for the test. Thus, for example, one would include a sufficient amount to provide the chemical interactions and changes necessary to enable the composition or device to be reactive with an indicator substance, in the presence of a particular peroxidatively active analyte, to an extent desired to achieve the sensitivity of response required in a particular analytical situation as well as the stability afforded by the presence of tetralin hydroperoxide. Accordingly, the amount of tetralin hydroperoxide can be widely varied, depending upon whether the analytical test is to be used for screening, semiquantitative or quantitative analysis for a particular analyte. The amount can be substantially the same as the amount of a conventional hydroperoxide, such as cumene hydroperoxide, used in heretofore known formulations, such as, for example, those disclosed in U.S. Patent Nos. 3,092,976 and 3,092,463, and in similar references which describe semiquantitative determinations of such analytes, and to which reference can also be made for preferred hydroperoxide concentration ranges. It is therefore

replacement of known organic hydroperoxides by tetralin hydroperoxide is possible in compositions and devices of the invention to achieve the stability advantages heretofore described, without materially affecting the functionality and sensitivity of the reagent system to a peroxidatively active analyte.

As previously indicated, the test composition of the invention which is improved by the presence of tetralin hydroperoxide comprises at least, in addition, an indicator compound which is capable, in the presence of the tetralin hydroperoxide and a peroxidatively active substance, of producing a detectable response, such as a color change or other response detectable visually or by instrumental means. Suitable indicators include the so-called "benzidine-type" compounds, for example, benzidine, *o*-tolidine, 3,3',5,5'-tetra-(lower alkyl) benzidine, 2,7-diaminofluorene or mixtures of these in varying proportions. By "lower alkyl" is meant an alkyl radical having 1 to 6 carbon atoms, including methyl, ethyl, *n*-propyl and isopropyl, and the various butyl, pentyl and hexyl isomers. Other suitable indicators include *o*-toluidine, *p*-toluidine, *o*-phenylenediamine, N,N'-dimethyl-*p*-phenylenediamine, N,N'-diethyl-*p*-phenylenediamine, *p*-anisidine, di-anisidine, *o*-cresol, *m*-cresol, *p*-cresol, alpha-naphthol, beta-naphthol, catechol, quaiacol, pyrogallol or those of the heterocyclic azine series, for example, bis-(N-ethyl-quinol-2-one)-azine or (N-methylbenzthiazol-2-one)-(1-ethyl-3-phenyl-5-methtriazol-2-one)-azine. However, most preferred as an indicator is 3,3',5,5'-tetramethylbenzidine.

In a preferred embodiment, the improved test composition of the invention is incorporated on or with a carrier matrix to form a solid phase, "dip-and-read" test device. Such a test device of the invention can be prepared by various well-known methods, which include impregnating an absorbent matrix material with a solution or solutions of the test composition and thereafter drying the impregnated matrix, thus incorporating within the matrix a finely divided, intimate mixture of the composition ingredients. Suitable carrier matrices which can be used include paper, cellulose, wood, synthetic resin fleeces, glass fiber paper, polypropylene felt, nonwoven or woven fabrics and the like. Most suitable and preferred for use in the present invention as a carrier matrix is a bibulous paper such as filter paper. It is to be appreciated, however, that selection of an appropriate material for use as a carrier matrix is a matter of routine choice for one skilled in the art, and that the matrix can take on various physical forms, all of which are intended as being within the scope of the present invention. The most preferred mode of preparation of the device is to impregnate the matrix in a one-step process wherein the ingredients of the composition are mixed together in a solution or suspension and the matrix is immersed, or dipped, into the solution, and thereafter removed and dried. The dried, impregnated matrix can then be affixed, by suitable means such as double-sided adhesive tape, to one end of a carrier member comprising, for example, an oblong plastic strip, the other end of the strip serving as a handle for ease of use.

In addition to the previously-described test composition ingredients which actively participate in the test reaction, further ingredients such as solvents to suspend the indicator used, thickening agents, wetting agents, buffers, emulsifying agents and other well known adjuvants can also be included in the test composition and device of the present invention. Thus, for example, as thickening agents various materials such as gelatin, algin, carrageenin, casein, albumin, methyl cellulose, polyvinylpyrrolidone and the like can be used. As a wetting agent, sodium laurylsulfate, is preferred, but any long chained organic sulfate or sulfonate, such as diocyl sodium sulfosuccinate or sodium dodecylbenzidine sulphonate can also be used. For the buffer systems, there can be used tartrate, phosphate, phthalate, citrate, acetate, or succinate buffers. Preferably, the compositions are buffered to a pH value of from about 3.5 to 7.0. As emulsifying agents there can be used polyvinyl alcohol, gum arabic, carboxy vinyl polymers and the like. The organic solvents which are useful to suspend the indicator include most nonreactive, volatile solvents such dimethylformamide, chloroform, ethylene dichloride, benzene, ethyl acetate and the like.

In use, the impregnated matrix of the test device can be immersed in a sample fluid or liquid suspension of the material to be tested and immediately withdrawn. In the presence of a peroxidatively active substance, for example, hemoglobin, contact of the test composition with the sample gives a positive, detectable response, e.g., a color reaction. The color response can then be compared, for example, with precalibrated color standards to obtain a semiquantitative estimation of the amount of hemoglobin contained in the sample. Intact red blood

cells may appear as dots or flecks of color on the otherwise uncolored matrix. Hemolyzed red blood cells may uniformly color the matrix. Other peroxidatively active substances detectable by compositions and devices of the invention include, for example, peroxidase, myoblobin, erythrocytes, and other *pseudoperoxidases*. In addition to visual comparison, various instrumental means can also be used to determine the color or other response in the matrix, thereby increasing the quantitation and accuracy of the test by obviating the subjective determination of the human eye.

It is to be appreciated that the composition and device of the invention can also be used to determine peroxidatively active substances not only in liquids but in solid or semi-solid substances such as feces, gastrointestinal contents and the like. Thus, for example, a thin layer of the solid or semi-solid substance can be applied to the carrier matrix of the device and the detectable response, such as color change, observed in the matrix.

The improved test composition and device of the invention have been found to be not only advantageous in terms of enhanced stability, but also highly sensitive to peroxidatively active substances, producing a detectable response to relatively low concentration thereof. In a preferred embodiment, the instant test composition has been found capable of detecting hemoglobin in urine at a concentration as low as 0.045 mg/dl, which corresponds to a blood dilution of 1:1,000,000. This high degree of sensitivity is at least commensurate with the sensitivity of many conventional "state-of-the-art" tests for peroxidatively active substances.

As described above, the composition of the present invention can be incorporated with a suitable carrier matrix in a variety of ways to form a test

device. For example, the ingredients can be dissolved or suspended in water or other suitable solvent, such as dimethylformamide, acetone, ethanol, or mixtures thereof. Such a solution or suspension can then be used to impregnate a filter paper carrier matrix. The carrier matrix can be dipped into or coated with the composition, for example with a doctor blade, or can be incorporated with the composition as an ink wherein the reagents are printed onto the matrix. The matrix is thereafter dried to form the finished device with the solid phase composition incorporated within the matrix.

The method presently preferred is to impregnate filter paper with a solution or suspension of the ingredients of the composition, the preferred solvents being distilled or deionized water and/or dimethylformamide (DMF). Such impregnation can be accomplished by dipping a piece of the filter paper into the solution or suspension and drying the paper in an air oven. To complete the device the dried paper is then cut into a square measuring about 0.6 centimeter (cm) on a side, which is mounted on one end of a polystyrene film strip measuring about 0.6 x 10 cm. Mounting is accomplished by use of double-sided adhesive transfer tape, such as that commercially available from the 3M Company.

Another method for making the device of the invention is one wherein an aqueous solution of some of the ingredients of the composition is impregnated into the filter paper matrix, and then an organic solution including the tetralin hydroperoxide and indicator is impregnated into the matrix. Thus, in this "two-dip" procedure, the filter paper is impregnated with the first solution, dried, reimpregnated with the second solution of the hydroperoxide and indicator, and dried a second time.

The most preferred method of making the device of the invention is to prepare a first aqueous solution of ingredients of the composition, comprising reagents (except for the hydroperoxide and indicator), buffers, wetting agents and the like, and then to prepare a second organic solution of the hydroperoxide and indicator reagents along with all remaining ingredients, followed by mixing of the two solutions, dipping of the filter paper in the mixture and drying. The device is then completed as previously described.

*EXAMPLE*

The following example is provided to further demonstrate the concepts and advantages of the present invention. The Example is intended to be illustrative of how to make and use the invention, and is not to be interpreted as limiting its scope in any way. Moreover, although a largely conventional organic synthesis procedure for preparing tetralin hydroperoxide is set forth in detail, it will be appreciated that this is also only by way of illustration. All percentages expressed herein are by weight, unless otherwise indicated.

a. <u>Synthesis of tetralin hydroperoxide</u>

Oxygen was bubbled through freshly distilled tetralin (163 grams (g), 1.21 mol) for 24 hours at 63-65°C. The temperature was then increased to 68-70°C and the reaction allowed to continue for an additional 24 hours. Excess starting material was removed by distillation in an argon atmosphere under reduced pressure (0.3 millimeters (mm)), and the oil which remained was recrystallized from

toluene-hexane. The crystals were washed with 50 milliliters (ml) of hexane and dried at a reduced pressure of 2 mm overnight in an argon atmosphere to produce 13.8 g (6.83% yield) of tetralin hydroperoxide, melting point 52.5-54°C. Iodometric titration showed the product to be 99% pure tetralin hydroperoxide.

b. <u>Preparation of the test composition and test devices</u>

A first solution was prepared by combining the following ingredients in the order listed:

25 ml distilled water
1.07 g sodium citrate; Buffer; 0.1 Molar (M)*,
1.39 g citric acid ; pH 6.9
3.34 g triethanolamine borate; 0.4 M*
0.5 g sodium laurylsulfate; 1.0%*
0.034 g ethylenediaminetetraacetic acid

A second solution was then prepared by combining the following:

25 ml dimethylformamide
0.2 ml 6-methoxyquinoline; 0.4%*
1.48 g tetralin hydroperoxide (prepared as previously described)
0.3 g 3,3',5,5'-tetramethylbenzidine; 0.03 M*
0.05 g orange G dye

The first solution was thoroughly mixed with the second solution to produce a test composition according to the invention having approximately a 0.2 Molar concentration of tetralin hydroperoxide.

To prepare a test device from the foregoing composition, a sheet of laboratory filter paper (Whatman 3MM) was impregnated with the composition by immersing it in the mixed solutions for about 8

---

*Final concentration of ingredient in mixed solutions

minutes, removing the paper and drying in an air oven at 105°C. A 0.6 centimeter (cm) square of the dried, impregnated paper was then cut and applied to one end of a polystyrene film strip, measuring about 0.6 x 10 cm, using a piece of double-sided adhesive transfer tape (3M Company).

Testing of devices of the invention produced as described above in urine samples containing various concentrations of hemoglobin, as low as 0.045 milligrams per deciliter, yielded visually discernible color levels corresponding to the hemoglobin concentrations, which indicated the ability of the devices to detect hemoglobin in urine. Control devices, which had been prepared substantially as previously described, except that cumene hydroperoxide was used therein rather than tetralin hydroperoxide, produced substantially identical color responses corresponding to the same hemoglobin levels in similar urine samples. The results of this testing indicated that the devices produced in accordance with this preferred embodiment of the invention were substantially as sensitive to hemoglobin in urine as the conventional devices which contained cumene hydroperoxide.

*STABILITY TESTING*

An experiment was conducted to determine the storage stability, i.e. "shelf-life" under ambient and elevated temperature conditions, of test devices which had been prepared according to the invention as described in the Example, *supra*.

A test composition, and test strip devices produced therefrom in accordance with the invention, were thus prepared as described in the Example and, in addition, control devices were prepared which were substantially identical to these devices, but which contained cumene hydroperoxide (2 ml, 0.2 Molar) rather than tetralin hydroperoxide. Following preparation, sets of the devices were "stressed", by storage for various periods of time ranging from one to four weeks, at 50°C. Storage for one week at 50°C was considered to correspond to storage for at least eight months at ambient temperature. After such storage, a set of the "stressed" devices was dipped in urine samples known to be negative in peroxidatively active substances, and produced no color response. Other sets of the "stressed" devices were dipped in urine samples to which had been added a known amount of human whole blood; color formation in these later devices was observed.

Color formation in additional sets of the devices was additionally monitored using a device known as a Macbeth Colorimeter (commercially available from Kollmorgan Company). This device is a microprocessor-controlled, scanning reflectance spectrophotometer suitable for the rapid measurement of reflectance spectra in the visual range. Measurements of the performance of reagent strip test devices in the Macbeth Colorimeter provide the following advantages over visual observation of color formation in the same strips:

1) the light source and the other conditions surrounding the sample remain fixed;

2) the detector characteristics remain fixed, whereas with visual observation the detector (i.e., the eyes of the observer) can vary from person to person, and with the same person, from day to day; and

3) instrumental measurement enables more precise quantitation of data than does visual observation and allows more objective comparisons to be made between results.

In order to meaningfully assess the data produced by the Macbeth Colorimeter, the results of the testing therewith were expressed in B* units. These units are an instrumental measure of the yellowness/-blueness value in a three dimensional color space. In order to obtain a B* value from a given reflectance (color) value produced by the Macbeth Colorimeter, a correlation is made by using the procedure described in D. B. Judd and G. Wyszeck:, *Color in Business, Science and Industry* (John Wiley & Sons, New York 1975). Thus, conventional test devices containing cumene hydroperoxide and not in accordance with the invention, which are "unstressed", i.e., considered substantially fully reactive, when immersed in urine samples containing 0.135 mg/dl hemoglobin visually produce a blue color; the corresponding correlated B* value is a small number (around 8). However, aged or otherwise "stressed" conventional devices, exhibiting a substantial loss of reactivity when dipped in urine containing 0.135 mg/dl hemoglobin, produce a green rather than blue color; such color correlates to a higher B* value (around 30). Accordingly, to determine the relative stability of test devices of the invention, the smaller the rate of change of B* with time under "stress" conditions, e.g., prolonged storage at elevated temperatures, the greater is the stability of the device.

Accordingly, Macbeth Colorimeter rate-of-change data obtained from a set of devices which had been produced according to the invention as described in the Example, *supra*, and data obtained from a set of control devices which had been produced as described

in the Example except that they contained cumene hydroperoxide rather than tetralin hydroperoxide, is set forth below. Readings were obtained from a set of the control and Example devices within 40-45 seconds after they had been removed from the aforesaid "stress" conditions, immersed in urine samples containing 0.135 mg/dl hemoglobin and removed; the readings were then compared with readings previously similarly obtained from other sets of the same devices. The rate-of-change of B* was then calculated. The average value of the rate-of-change of reactivity over all of the devices is set forth in the following table, in term of B* units per week.

| Device type | Rate-of-change (B*/week) |
|---|---|
| cumene hydroperoxide (control) | 27.51 |
| tetralin hydroperoxide | 13.42 |

Moreover, color formation, visually observed, was of substantially greater intensity and uniformity in the devices of the invention than in the control devices, after the "stress" periods.

The foregoing data shows that test devices of the invention which incorporated tetralin hydroperoxide exhibited substantially increased stability in reactivity to hemoglobin after adverse storage and temperature "stress" conditions, by comparison with conventional test devices which incorporated cumene hydroperoxide. Moreover, this enhanced stability, i.e., "shelf-life" of the devices of the invention, advantageously enabled them to satisfactorily detect a 0.135 mg/dl concentration of hemoglobin in urine even after such "stress". However, the degradation of reactivity of the conventional devices incorporating cumene hydroperoxide was comparatively so rapid and substantial as to render them, after the "stress" periods, much less reactive

to hemoglobin and thus considerably less desirable as an analytical tool after such prolonged and elevated temperature storage.

It is to be appreciated that many modifications and variations in the preferred embodiments of the instant invention as set forth herein are possible without departing from the spirit and scope of the invention, and that any limitations upon such spirit and scope are intended to be imposed only by the following claims.

WHAT IS CLAIMED IS:

1. A solid phase composition for the determination of a peroxidatively active substance in a test sample, the composition comprising an organic hydroperoxide and an indicator capable of providing a detectable response in the presence of said organic hydroperoxide and peroxidatively active substance, characterized in that said organic hydroperoxide is tetralin hydroperoxide.

2. A composition according to Claim 1, characterized in that the indicator comprises benzidine, o-tolidine, 3,3', 5,5'-tetra(lower alkyl)-benzidine, 2,7-diaminofluorene or mixtures thereof.

3. A test device for determining the presence of a peroxidatively active substance in a test sample comprising a carrier matrix incorporated with a composition according to Claim 1 or Claim 2.

4. A method for determining the presence of a peroxidatively active substance in a test sample, characterized in that it comprises the steps of contacting the sample with a device according to Claim 3 and observing a detectable response in the device.

5. A method for preparing a test device for determining the presence of a peroxidatively active substance in a test sample, comprising the steps of:

preparing a solution comprising an organic hydroperoxide and an indicator capable of providing a detectable response in the presence of the hydroperoxide and the peroxidatively active substance;

incorporating the solution with a carrier matrix by wetting the matrix with the solution; and

drying the wetted matrix to leave a residue of the composition therein, characterized in that the hydroperoxide used is tetralin hydroperoxide.

6. A method according to Claim 5, characterized in that it further comprises the additional step of affixing the dried matrix to a carrier member.